# EUROPEAN PATENT SPECIFICATION

(11) **EP 0 467 775 B1**
(45) Date of publication and mention of the grant of the patent: **10.04.1996**
(21) Application number: 91401985.6
(22) Date of filing: 16.07.1991
(51) Int. Cl.: A61F 9/00

(54) **Lens capsule laser cutting apparatus**
Lasereinrichtung zum Schneiden der Linsenkapsel
Capsulotome à laser

(30) Priority: 19.07.1990 JP 191446/90
(43) Date of publication of application: 22.01.1992
(73) Proprietor: Kabushiki Kaisha TOPCON, Tokyo (JP)
(72) Inventor: Kobayashi, Katsuhiko, Itabashi-ku, Tokyo 174 (JP)
(74) Representative: Poulin, Gérard

(56) References cited:
- EP-A- 0 402 250
- WO-A-87/05794
- FR-A- 2 458 272
- US-A- 3 348 547
- US-A- 3 971 382
- US-A- 4 887 592
- CONFERENCE ON LASERS AND ELECTRO-OPTICS, Anaheim, CA, 25th - 29th April 1988, vol. 7, page 7, no. THM51, Washington, DC, US; M. CHILBERT et al.: "Lensectomy procedure using an endoexcimer laser system"

## Description

### INDUSTRIAL FIELD OF APPLICATION

This invention concerns an apparatus for performing ophthalmic surgery, and in particular, a lens capsule laser cutting apparatus suitable for removing the anterior lens capsule in extracapsular cataracy extractions.

### BACKGROUND OF THE INVENTION

Cataracts are a disease where the lens nucleus becomes cloudy whitish grey, and visual power declines as a result. Cataract surgery involves removal of the nucleus.

Cataract surgeries are presently performed by means of the following 3 techniques.
(1) Intracapsular cataract extraction
(2) Extracapsular cataract extraction (wherein an incision is made in the anterior lens capsule by a special scalpel formed by transforming a hypodermic needle, the anterior lens capsule is exposed to ultrasonic waves, and the lens nucleus is aspirated after emulsification), and
(3) Ultrasonic cataract operations (wherein both the anterior lens capsule and lens nucleus are aspirated after emulsification by ultrasonic waves).

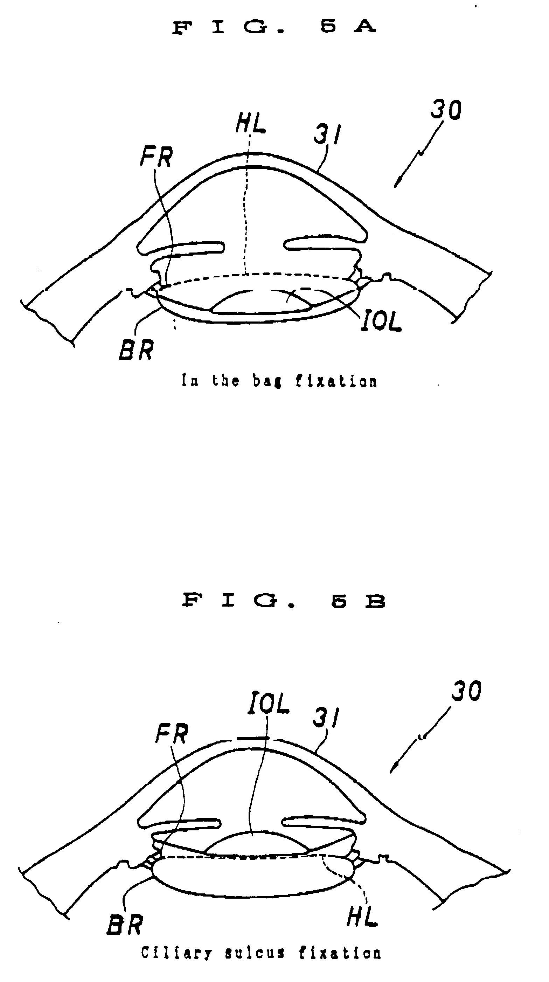

In (1), the crystallin is extracted together with the capsule. In (2) and (3), the posterior lens capsule BR is left as shown in Fig. 5A and 5B, and only part of the anterior lens capsule FR, lens capsule and lens are removed.

Recently, it has become frequent to insert an intraocular lens (IOL) after removing the lens nucleus by method (1), (2) or (3). It is desirable to insert the IOL in the posterior chamber, and also preferable from the safety viewpoint to fix it in the capsule after insertion (Fig. 5A). Methods (2) and (3) are becoming increasingly common as they are better adapted to this posterior chamber lens.

The recent consensus of academic opinion is that method (2) is far superior to method (3) (and that method (3) is far more dangerous). There is one way of performing (2) known as Continuous Circular Capsulorhexis (C.C.C.) wherein an annular incision is made in the anterior lens capsule. This is now the most usual technique for the following reasons:
(1) The incision on the anterior lens capsule is symmetrical,
(2) The rim of the anterior lens capsule does not tear after IOL insertion,
(3) There is little damage to ocular tissues after IOL insertion,
(4) As the IOL is stable, preferable visual acuity is maintained.

The success or failure of the C.C.C. technique however depends on (1) how close the incision in the anterior lens capsule is to a perfect circle (2) how clean the incision is, and (3) whether the incision can be made without damaging other ocular tissues. These factors depend largely on the experience and skill of the surgion. If the incision is made without satisfying conditions (1) - (3), the zonules of Zinn and lens capsules would be damaged, and it would also have an adverse effect on the set of procedures carried out subsequent to the incision such as extraction of the lens nucleus (phacoemulsification and aspiration), aspiration of the cortex, and insertion of the intraocular lens. The above conditions are therefore crucial to the success of the operation in the early or late post-operative results.

If the operator is not sufficiently experienced, the capsule may tear when the incision in the anterior lens capsule is made. Further, if the anterior lens capsule incision is asymmetrical (not circular), or if the incision is circular but has a zig-zag edge, uneven stresses are set up in the following steps, i.e. extraction of the lens nucleus (phacoemulsification and aspiration), aspiration of the cortex and insertion of the intraocular lens (IOL). The lens capsule may therefore tear, and the zonules of Zinn may be damaged. Further, if the anterior lens capsule incision is asymmetrical, the inserted IOL is sometimes found not to coincide with the ocular optical axis after the surgery.

This invention therefore aims to provide a lens capsule laser cutting apparatus which improves the post-operative result of cataract surgery, and permits the C.C.C. technique to be performed accurately and easily without the slightest damage to other ocular tissues.

FR-A-2 458 272 discloses a lens capsule cutting apparatus according to the preamble of claim 1.

In order to achieve the above objectives, the lens capsule cutting apparatus of this invention is in accordance with claim 1.

Said converging means preferably consists of one focusing lens. Further, it is desirable that said axicon means comprises at least one axicon lens capable of moving along the optical path of said projecting means.

Said projecting means should preferably comprise an expander means to expand the radius of the laser beam generated by said generating means.

The source is preferably an infrared pulse laser with a wavelength of approximate 1.06 µm, but a Dye laser may also be used.

The apparatus also includes an aiming means for projecting a visible ring-like laser beam on the lens capsule which are substantially coincident with the laser beam projected by said axicon means.

The optical path of said aiming means preferably overlaps with a part of the optical path of said projecting means. Preferably, the aiming means comprises a visible laser beam source, and a mirror obliquely interposed between said beam expander means and said converging means for reflecting the visible laser beam and allowing the laser beam from the generating means to pass therethrough.

By means of the lens capsule laser cutting apparatus of this invention, a lens capsule can be cut out in a perfect circle with a ring-like laser beam. As the laser cutting apparatus involves no contact with ocular tissues, moreover, the incision can be made without applying great effort, and there can be little damage to the zonules of Zinn or other ocular tissues.

Using this apparatus, the annular incision on the lens capsule can be made uniform and continuous.

Uneven stresses on the incision are therefore reduced in subsequent operative procedures such as phacoemalsificate aspiration of the lens nucleus, aspiration of the lens cortex and insertion of the IOL, and there is less risk of tearing the lens capsule and thereby damaging the zonules of Zinn. This leads to better results in the early and later post-operative results.

Further, as the incision in the anterior lens capsule can be clean, the IOL inserted in the capsule can be maintained exactly at the ocular optical axis after the surgery.

Further, no special training is required to perform the incision, and the operation can be carried out in less time. Moreover, by focusing the laser beam on the posterior lens capsule, it is also possible to perform after-cataract surgery.

### BRIEF DESCRIPTION OF THE DRAWINGS

Fig. 1 is a drawing showing the optical arrangement of a first embodiment of the lens capsule laser cutting apparatus of this invention.

Fig. 2 is a partial optical drawing of a second embodiment of the lens capsule laser cutting apparatus of this invention.

Fig. 3 is a partial optical drawing of a third embodiment of the lens capsule laser cutting apparatus of this invention.

Fig. 4 is a drawing showing the optical arrangement of a modification of the first embodiment.

Figs 5A and 5B are schematic drawings showing the way in which an IOL is inserted into the posterior chamber.

### DETAILED DESCRIPTION OF THE PREFERRED EMBODIMENTS

The preferred embodiments of this invention shall be described in more detail with reference to the examples shown in the attached drawings.

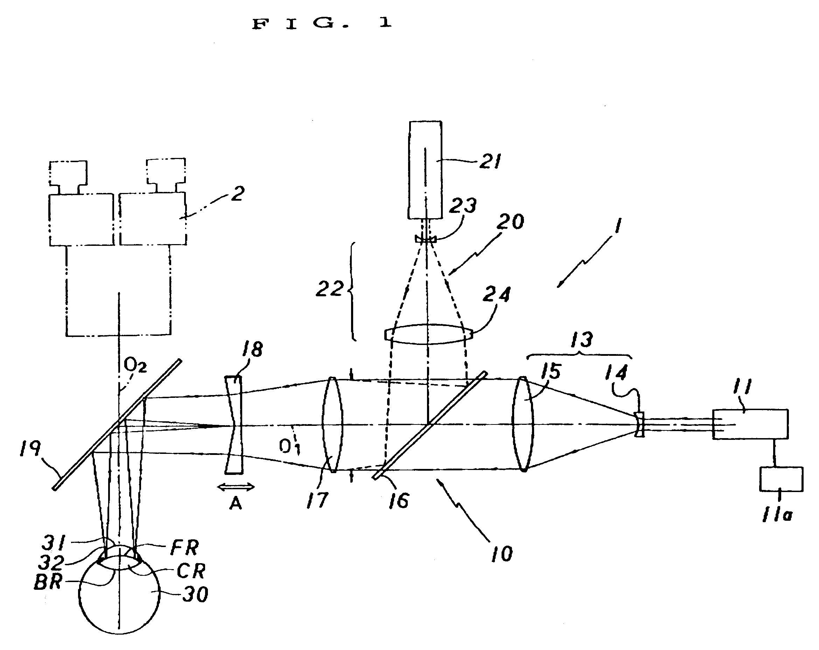

As shown in Fig. 1, projecting means 1 of the lens capsule laser cutting apparatus of this invention comprises an optical system 10 for projecting a laser beam and an aiming optical system 20.

The projecting optical system 10 comprises a laser source 11 generating a laser beam capable of ablating the tissue of living organisms or a lens capsule. Lasers which satisfy this condition include for example Nd:YAG and Dye. The wavelengths of these laser beam lie in the approximate range 0.4 µm - 1.5 µm which is not absorbed by water, the principal constituent of the cornea and aqueous humor, and a wavelength of approximate 1.06 µm is preferable. The laser source is therefore an Nd:YAG laser. Further, it is desirable that the laser power density on the lens capsule is at least 10⁸W/cm² in order that the capsule is not evaporated by absorbing laser energy, but is destroyed by the laser's optical breakdown effect.

The Nd:YAG laser 11 is connected to a radiation control switch 11a. When this switch is turned on, the laser source generates an infrared pulse laser beam.

The output laser beam from the Nd:YAG laser source 11 is enlarged in radius through a beam expander 13 which comprises a concave lens 14 and a convex lens 15. The laser beam from the convex lens 15 is made parallel, and is projected in turn toward a diverging prismatic lens (axicon lens) 18 through a condensing (focusing) lens 17 and a cold mirror 16. The axicon lens 18 has a prism-like reverse conical shape in sectional view, and the outer edge thereof (prism base) is larger in width than the optical axis portion thereof. Due to the prism function of the axicon lens 18, the output beam therefrom is made ring-like. The ring-like beam is then reflected by a dichroic mirror 19, and converged on the anterior lens capsule FR of a crystallin lens CR to be treated through the cornea 31 of a patient's eye 30.

As a result, the anterior lens capsule FR is cut out in a ring shape by the energy of the laser beam, and a circular hole HL (Figs. 5A, 5B) is obtained in the anterior lens capsule FR.

To induce an optical breakdown in the anterior lens capsule FR, it is required to set the width of the ring-like laser beam to be narrow. and the numerical aperture of the focusing lens 17 must therefore be large. The beam expander 13 contributes to enlarging the numerical aperture of lens 17 by increasing the radius of the ring-like incident laser beam.

In order to obtain a hole HL of desired diameter in the anterior lens capsule FR (normally 6 - 6.5 mm), it is necessary to vary the diameter of the ring-like laser beam 32 projected on the anterior lens capsule FR. For thin purpose, the axicon lens 18 is preferably mounted so that it is movable along the optical axis O₁ in the direction of the arrow A.

The aiming optical system 20 (see Fig. 1) comprises a He-Ne laser source 21, a beam expander 22 and the cold mirror 16. The He-Ne laser source 21 generates a visible laser beam. The beam expander 22 comprises a concave lens 23 and a convex lens 24. The cold mirror 16 reflects only incident He-Ne laser beams, while Nd:YAG laser beams pass therethrough.

The Ne-He laser beam from the laser source 21 is enlarged in diameter by the expander 22 from which the output laser beam is in turn reflected by the cold mirror 16 and projected to the condensing (focusing) lens 17. The He-Ne laser beam from the condensing lens 17 therefore travels along the same optical path as the Nd:YAG laser beam.

To observe the anterior lens capsule FR of the lens CR and the ring-like He-Ne laser beam projected thereon, this laser cutting apparatus is provided with an operation microscope 2 as indicated by the phantom lines in Fig. 1. This operation microscope 2 is well known in the ophthalmology field, and its detailed explanation is therefore omitted.

The optical axis O₂ of the operation microscope 2 is adjusted to be coincident with that of the laser cutting apparatus O₁. The dichroic mirror 19 functions as a half mirror for the He-Ne laser beam, but as a full reflecting mirror for the Nd:YAG laser beam. A surgeon can thus determine the optimum diameter of the hole HL to be created in the anterior lens capsule FR of lens CR by observing the He-Ne laser beam projected thereon through the operation microscope 2.

An annular incision can be made in the anterior lens capsule FR using this laser cutting apparatus as described below.

Visible aiming laser beam from the He-Ne laser source 21 is projected on the anterior lens capsule FR of a patient's eye through the beam expander 22, cold mirror 16, focusing lens 17, (condensing lens 17), axicon lens 18 and dichroic mirror 19.

The visible aiming laser beam projected on the anterior lens capsule FR is ring-like in shape, and it can be observed through the operation microscope 2. The focus of the projected laser beam may be adjusted by moving the operation microscope and laser cutting apparatus along the optical axis O₂ together with each other.

Next, the axicon lens 18 is moved along the optical axis O₁ so that the diameter of the ring-like laser beam 32 is optimum. After the diameter of the ring-like laser beam 32 has been set to optimum by means of the operation microscope 2 and aiming system 20, the control switch 11a is turned on and the Nd:YAG laser source 11 generates an invisible Nd:YAG pulse laser beam. The Nd:YAG laser beam which is to make an incision on the anterior lens capsule FR of lens CR is then projected by the beam expander 13, cold mirror 16, condensing lens 17, axicon lens 18 and dichroic mirror 19.

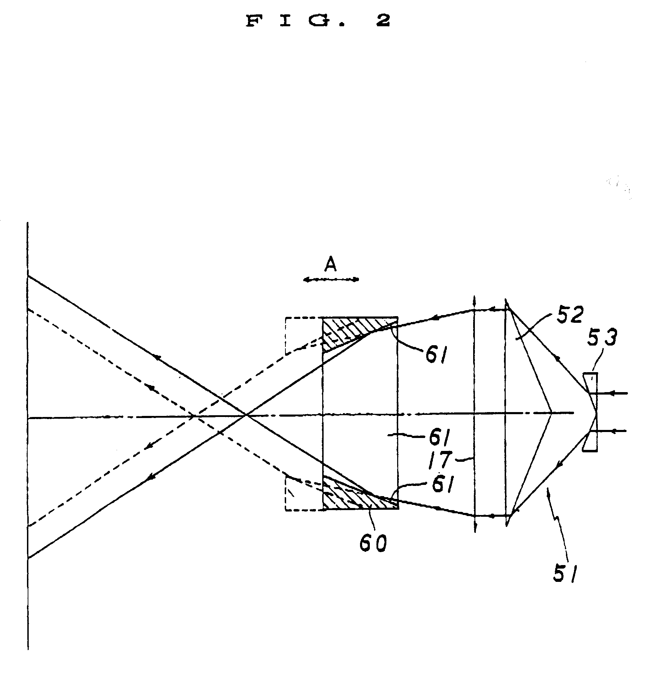

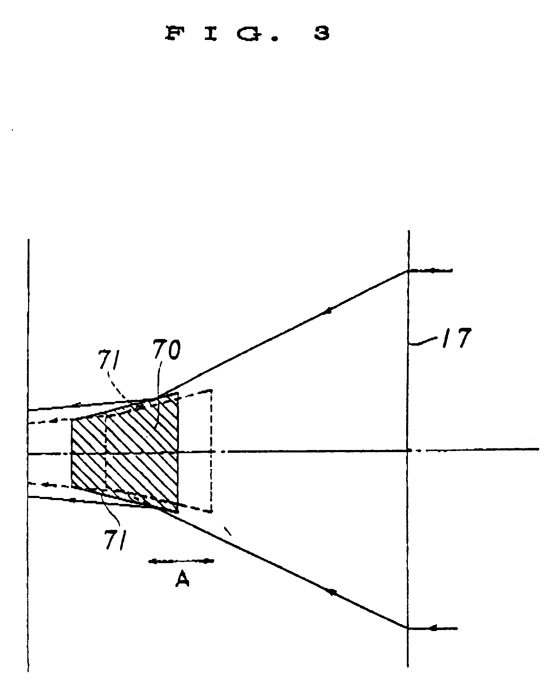

Figs. 2 and 3 respectively show second and third embodiments of the lens capsule laser cutting apparatus of this invention.

The optical system of the second embodiment comprises a fixed beam expander 51, focusing lens 17 and axicon mirror 60 which can be moved along the optical axis thereof. In this embodiment, the fixed beam expander 51 is directly connected optically to a laser beam source 11 not shown. The beam expander 51 performs a similar function to the beam expander 13 shown in Fig. 1. It is moreover preferable that the cold mirror 16 is disposed between the laser source 11 and fixed beam expander 51.

Alternatively, the cold mirror 16 may be interposed between the fixed beam expander 51 and focusing lens 17. The fixed beam expander 51 comprises a diverging axicon lens 53 and a converging axicon lens 52. The movable axicon mirror 60 has an inner mirror surface 61. In response to its movement along the optical axis in the direction of arrow A, the diameter of the annular incision varies.

The third embodiment shown in Fig. 3 has an outer mirror surface 71 on a movable axicon mirror 70 which can be adjusted in an axial direction thereof. The axicon mirror 70 is positioned in place of the axicon lens 18 shown in Fig. 1, and performs substantially the same function as the latter.

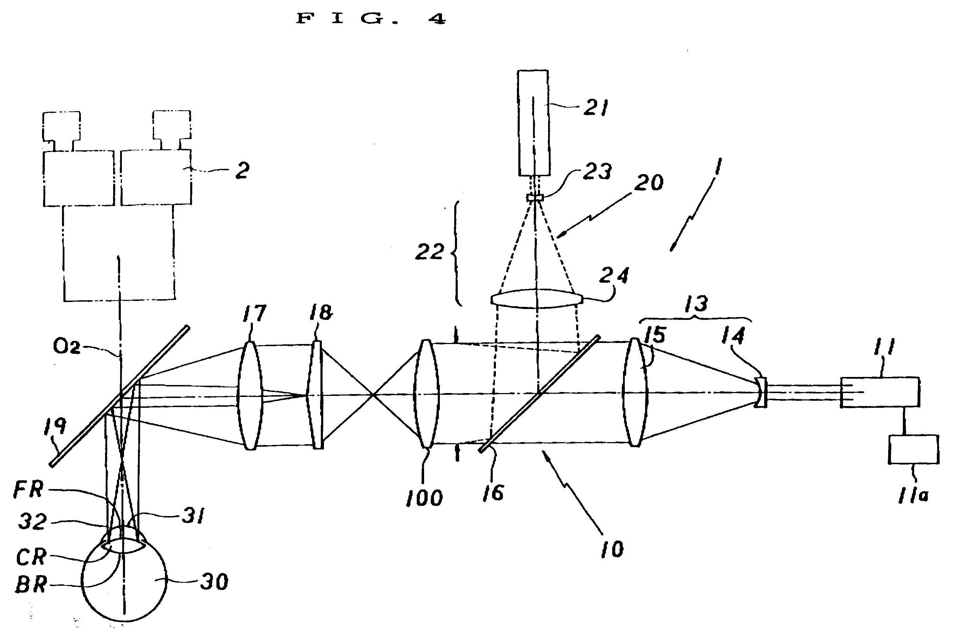

Fig. 4 shows a further modification of the first embodiment (Fig. 1). If a relay lens 100 is added, a converging (focusing) lens 17 may also be disposed between the axicon lens 18 (which is of the converging type in this embodiment) and the dichroic mirror 19. The relay lens 100 makes an intermediate image 101 of the laser beam from the beam expander 13 at its focal point situated before the axicon lens 18. The focusing lens 17 situated after the axicon lens 18 converges the laser beam from the axicon lens onto the lens capsule.

In these embodiments a Nd:YAG laser is respectively used, but a visible pulse laser such as a Dye laser may also be used as another optical source.

The present embodiment is to be considered in all respects as illustrative and not restrictive, the scope of the invention being indicated by the appended claims rather than by the foregoing description, and all changes which come within the meaning of the claims are therefore intended to be embraced therein.

## Claims

1. A lens capsule cutting apparatus comprising :
means for generating an infrared pulse laser beam having a wavelength of 0.4 µm to 1.5 µm ; and
means for projecting the laser beam onto a lens capsule for cutting said capsule ;
said projecting means incluing a converging optical means to converge the laser beam, characterized in that said projecting means also includes an axicon lens means for projecting the converged beam in an annular shape onto said capsule and varying the diameter of the beam ;
the converged laser beam on said lens capsule having a power density of no less than 10⁸ W/cm².

2. A lens capsule cutting apparatus as defined in Claim 1 wherein said axicon means can be moved along an optical axis of said projecting means.

3. A lens capsule cutting apparatus as defined in claim 2 wherein said projecting means further comprises a beam expander to enlarge an energing radius of the laser beam emerging from said generating means, wherein said converging optical means converges the laser beam from said beam expander through said axicon lens means onto said lens capsule.

4. A lens capsule cutting apparatus as defined in claim 2 wherein said projecting means further comprises a beam expander to enlarge a beam radius of the laser beam and an image forming optical means to form an intermediate image of said laser beam from said beam expander at an intermediate point situated before said axicon lens means, wherein said converging optical means is situated after said axicon lens means for converging said laser beam from said axicon lens means onto said lens capsule.

5. A lens capsule cutting apparatus as defined in Claim 1 wherein the wavelength of the beam generated by said infrared pulse beam generator is 1.06 µm.

6. A lens capsule cutting apparatus as defined in claim 3 further comprising an aiming means for projecting a visible annularly shaped laser beam coincident with the laser beam projected by said axicon lens means on the lens capsule, in order to see a visible laser beam spot thereon.

7. A lens capsule cutting apparatus as defined in claim 6 wherein an optical axis of said aiming means overlaps with part of the optical axis of said projecting means.

8. A lens capsule cutting apparatus as defined in claim 7 wherein said aiming means comprises a visible laser beam source and a mirror which reflects the visible laser beam while allowing the laser beam from said generating means to pass therethough, said mirror being positioned obliquely between said beam expander means and said converging optical means.

9. A lens capsule cutting aparatus as defined in claim 5 wherein said generating means is a Nd:YAG laser optical source.

10. A lens capsule cutting apparatus as defined in claim 1 wherein said generating means is a Dye laser optical source.

11. A lens capsule cutting apparatus according to claim 3 wherein said beam expander comprises a variable diverging beam expander.

## Patentansprüche

1. Vorrichtung zum Schneiden der Linsenkapsel, die enthält
eine Vorrichtung zur Erzeugung eines infraroten gepulsten Laserstrahls, der ein Wellenlänge zwischen 0,4 µm und 1,5 µm besitzt;
eine Vorrichtung zur Projektion des Laserstrahls auf eine Linsenkapsel, um die genannte Kapsel zu schneiden;
wobei die genannte Projektionsvorrichtung eine optische Bündelungsvorrichtung zur Bündelung des Laserstrahls enthält, dadurch gekennzeichnet, daß die genannte Projektionsvorrichtung auch eine Axicon-Linsenvorrichtung zur Projektion des gebündelten Strahls in einer ringförmigen Gestalt auf die genannte Kapsel und zur Veränderung des Strahldurchmessers besitzt;
wobei der gebündelte Laserstrahl auf der genannten Linsenkapsel eine Energiedichte nicht geringer als 10⁸ W/cm² besitzt.

2. Vorrichtung zum Schneiden der Linsenkapsel nach Anspruch 1, wobei die genannte Axicon-Vorrichtung entlang einer optischen Achse der genannten Projektionsvorrichtung bewegbar ist.

3. Vorrichtung zum Schneiden der Linsenkapsel nach Anspruch 2, wobei die genannte Projektionsvorrichtung weiterhin einen Strahlaufweiter zur Vergrößerung eines Strahlradius des Laserstrahls umfaßt, der aus der genannten Erzeugungsvorrichtung austritt, wobei die genannte optische Bündelungsvorrichtung den Laserstrahl von dem genannten Strahlaufweiter durch die genannte Axicon-Linsenvorrichtung auf die genannte Linsenkapsel bündelt.

4. Vorrichtung zum Schneiden der Linsenkapsel nach Anspruch 2, wobei die genannte Projektionsvorrichtung weiterhin einen Strahlaufweiter zur Vergrößerung eines Strahlradius des Laserstrahls und eine bilderzeugende, optische Vorrichtung zur Erzeugung eines Zwischenbildes des genannten Laserstrahls von dem genannten Strahlaufweiter an einem Zwischenpunkt umfaßt, der sich vor der genannten Axicon-Linsenvorrichtung befindet, wobei die genannte optische Bündelungsvorrichtung nach der genannten Axicon-Linsenvorrichtung zur Bündelung des genannten Laserstrahls von der genannten Axicon-Linsenvorrichtung auf die genannten Linsenkapsel angeordnet ist.

5. Vorrichtung zum Schneiden der Linsenkapsel nach Anspruch 1, wobei die Wellenlänge des durch den genannten Erzeuger eines gepulsten infraroten Strahls erzeugten Strahles 1,06 µm beträgt.

6. Vorrichtung zum Schneiden der Linsenkapsel nach Anspruch 3, die weiterhin eine Zielvorrichtung zur Projektion eines sichtbaren ringförmig ausgebildeten Laserstrahls enthält, der koinzident mit dem Laserstrahl ist, der durch die genannte Axicon-Linsenvorrichtung auf die Linsenkapsel projiziert wird, um darauf einen Ort eines sichtbaren Laserstrahls zu sehen.

7. Vorrichtung zum Schneiden der Linsenkapsel nach Anspruch 6, wobei eine optische Achse der genannten Zielvorrichtung mit einem Teil der optischen Achse der genannten Projektionsvorrichtung überlappt.

8. Vorrichtung zum Schneiden der Linsenkapsel nach Anspruch 7, wobei die genannte Zielvorrichtung eine Quelle für einen sichtbaren Laserstrahl und einen Spiegel umfaßt, der den sichtbaren Laserstrahl reflektiert, während er den Laserstrahl von der genannten Erzeugungsvorrichtung durchgehen läßt, wobei der genannte Spiegel schräg zwischen der genannten Strahlaufweitungsvorrichtung und der genannten optischen Bündelungsvorrichtung angeordnet ist.

9. Vorrichtung zum Schneiden der Linsenkapsel nach Anspruch 5, wobei die genannte Erzeugungsvorrichtung eine optische Nd:YAG-Laserquelle ist.

10. Vorrichtung zum Schneiden der Linsenkapsel nach Anspruch 1, wobei die genannte Erzeugungsvorrichtung eine optische Farbstofflaserquelle ist.

11. Vorrichtung zum Schneiden der Linsenkapsel nach Anspruch 3, wobei der genannte Strahlaufweiter einen veränderlichen divergierenden Strahlaufweiter umfaßt.

## Revendications

1. Appareil de capsulectomie, comprenant :
des moyens générateurs d'un faisceau laser pulsé infrarouge ayant une longueur d'onde de 0,4 µm à 1,5 µm ; et
des moyens pour projeter le faisceau laser sur une capsule de cristallin afin de découper ladite capsule ;
lesdits moyens de projection comprenant un moyen optique convergent, pour faire converger le faisceau laser, caractérisé en ce que lesdits moyens de projection comprennent également un moyen de lentille axicon pour projeter le faisceau qui converge sous une forme annulaire sur ladite capsule et pour modifier le diamètre du faisceau ;
le faisceau laser qui converge sur ladite capsule du cristallin ayant une densité de puissance non inférieure à 10⁸ W/cm².

2. Appareil de capsulectomie selon la revendication 1, dans lequel ledit moyen axicon peut être déplacé suivant un axe optique desdits moyens de projection.

3. Appareil de capsulectomie selon la revendication 2, dans lequel lesdits moyens de projection comprennent en outre un dilatateur de faisceau pour agrandir un rayon du faisceau laser sortant desdits moyens générateurs, dans lequel ledit moyen optique convergent fait converger le faisceau laser, venant dudit dilatateur de faisceau, par l'intermédiaire dudit moyen de lentille axicon, sur ladite capsule du cristallin.

4. Appareil de capsulectomie selon la revendication 2, dans lequel lesdits moyens de projection comprennent en outre un dilatateur de faisceau pour agrandir un rayon de faisceau du faisceau laser, et un moyen optique de formation d'image servant à former une image intermédiaire dudit faisceau laser, venant dudit dilatateur de faisceau, en un point intermédiaire situé avant ledit moyen de lentille axicon, dans lequel ledit moyen optique convergent est situé après ledit moyen de lentille axicon pour faire converger ledit faisceau laser venant dudit moyen formant lentille axicon sur ladite capsule du cristallin.

5. Appareil de capsulectomie selon la revendication 1, dans lequel la longueur d'onde du faisceau, produit par ledit générateur de faisceau pulsé infrarouge, est de 1,06 µm.

6. Appareil de capsulectomie selon la revendication 3, comprenant en outre moyen de visée pour projeter un faisceau laser visible de forme annulaire, qui coïncide avec le faisceau laser projeté par ledit moyen de lentille axicon, sur la capsule du cristallin, afin de voir, sur celle-ci, une tache du faisceau laser visible.

7. Appareil de capsulectomie selon la revendication 6, dans lequel un axe optique desdits moyens de visée recouvre une partie de l'axe optique desdits moyens de projection.

8. Appareil de capsulectomie selon la revendication 7, dans lequel lesdits moyens de visée comprennent une source de faisceau laser visible et un miroir, qui réfléchit le faisceau laser visible tout en laissant passer à travers lui le faisceau laser venant desdits moyens générateurs, ledit miroir étant disposé obliquement entre ledit moyen dilatateur de faisceau et ledit moyen optique convergent.

9. Appareil de capsulectomie selon la revendication 5, dans lequel lesdits moyens générateurs sont une source optique de laser YAG au néodyme.

10. Appareil de capsulectomie selon la revendication 1, dans lequel lesdits moyens générateurs sont une source optique de laser à colorant.

11. Appareil de capsulectomie selon la revendication 3, dans lequel ledit dilatateur de faisceau comprend un dilatateur de faisceau divergent variable.
